# EUROPEAN PATENT SPECIFICATION

(11) **EP 3 662 875 B1**
(45) Date of publication and mention of the grant of the patent: **02.10.2024**
(21) Application number: 18850672.9
(22) Date of filing: 29.08.2018
(51) Int. Cl.: A61F 9/007

(54) **BACKFLUSH NEEDLE**
RÜCKSPÜLNADEL
AIGUILLE DE RINÇAGE

(30) Priority: 30.08.2017 JP 2017165799
(43) Date of publication of application: 10.06.2020
(73) Proprietor: Mani, Inc., Tochigi 321-3231 (JP)
(72) Inventor: MURAKAMI, Etsuo, Utsunomiya-shi Tochigi 321-3231 (JP); TAKANO, Masahiro, Utsunomiya-shi Tochigi 321-3231 (JP)
(74) Representative: Isarpatent
(86) International application number: PCT/JP2018/031900
(87) International publication number: WO 2019/044884

(56) References cited:
- CA-A1- 2 960 278
- DE-A1- 3 434 930
- JP-A- 2015 016 203
- US-A1- 2006 229 563
- US-A1- 2012 041 369
- US-A1- 2012 283 741
- US-A1- 2015 173 947

## Description

### Technical Field

The present invention relates to a backflush needle used in vitreous surgery.

### Background Art

CA 2 960 278 A1 (PSIVIDA INC [US]), 17 March 2016 (2016-03-17), relates to an injector apparatus for delivering a payload into a tissue, and discloses an injector device for delivering a pay load to a tissue, comprising a housing, a cannula defining a lumen and having a proximal end received in the housing and a distal end extending from the housing, a hollow needle comprising a distal end with a tip for piercing tissue, a shifter that shifts the needle from an extended position to a retracted position, and an actuator for advancing a pay load from an initial position to exit from the distal end of the cannula. In the extended position, the needle occupies at least part of the lumen of the cannula, and the distal end of the needle extends past the distal end of the cannula, and in the retracted position, the distal end of the needle is withdrawn from the lumen of the cannula sufficiently to permit a pay load to advance from an initial position through the distal end of the cannula.
US 2012/041369 A1 (BECKER BRUCE [US]), 16 February 2012 (2012-02-16), relates to a retrobulbar needle part of a syringe.
US 2012/283741 A1 (LULOH K PETER [US] ET AL), 8 November 2012 (2012-11-08), relates to a retractable tip for a vitrectomy tool.
DE 34 34 930 A1 (GEUDER HANS GMBH [DE]), 3 April 1986 (1986-04-03), relates also to an incision tool for the eye.
Vitreous surgery performed by an ophthalmologist is surgery for aspirating and removing a gelatinous vitreous body within an eyeball, a proliferative membrane generated on the retina through degeneration of the vitreous body, blood, and/ or impurities etc. A backflush needle is used as a surgical tool used in such a vitreous surgery (see Patent Document 1, for example).

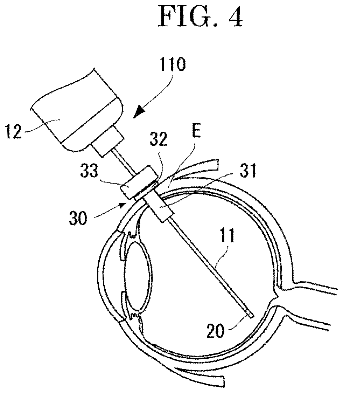

FIG. 4 is a use explanation drawing of the backflush needle. A backflush needle 110 has a small, tubular needle main body 11 to be connected to a handle 12, which is for a surgeon to grip, and inserted in an eyeball, and a soft member 20 to be connected to the tip of the needle main body 11. The soft member 20 is provided such that a front end of the tool does not damage the retina during surgery even when it touches the retina, and provision of the soft member 20 allows aspiration of a vitreous body etc. near the retina.

Connection between the needle main body 11 and the soft member 20 is carried out using an adhesive, and a connecting portion is made to have a fittable structure. Note that while the material of the soft member 20 is not limited, silicone resin may be used, for example, and it may have a shape like a brush, not limiting the shape to a tubular form.

The backflush needle 110 is used, passed through the cannula 30 that is attached to an eyeball E. The cannula 30 has a metal cannula pipe 31 fit in a cannula base 32 made of resin. The cannula pipe 31 is thrust into a sclera when attaching the cannula 30 to the eyeball E, wherein the cannula base 32 functions as a stopper so as to keep from thrusting in too far at this time. Moreover, in order to control leakage of vitreous humor from the inside of the eyeball via the cannula pipe 31 when the cannula 30 has been attached to the eyeball E, a cannula cap 33 covering from the cannula base 32 to a back end of the cannula pipe 31 may be used. Note that the cannula cap 33 is provided with a slit through which an ophthalmic operation tool, such as the backflush needle 100, is passed.

When passing the backflush needle 110 through the cannula 30, the soft member 20 on the tip touches the slit of the cannula cap 33 and the hollow cavity inner surface of the cannula pipe 32. This may deform the soft member 20, and the connecting portion of the soft member 20 and the needle main body 11 may come off. Deformation of the soft member 20 may be a cause of inhibiting aspiration of the vitreous body etc., and if the soft member 20 detaches from the needle main body 11, the soft member 20 becomes difficult to pull out from the eyeball.

### Prior Art Documents

### Patent Documents

[Patent Document 1] JP 2014-50433A

### DISCLOSURE OF INVENTION

### Problem to be Solved by the Invention

In light of this problem, the present invention aims to provide a backflush needle that can keep the shape of a soft member on the tip when passed through a cannula.

### Solution to the Problem

A backflush needle of the present invention for achieving the above aim is used in an ophthalmic operation with the backflush needle passed through a cannula attached to an eyeball. The needle includes a tubular needle main body to pass through the cannula, a tubular soft member formed from silicon resin connected to a front end of the needle main body, and a rod-shaped core member to pass through respective tubular hollow cavities of the needle main body and the soft member. The core member is axially movable, allowing protrusion of a front end of the core member from the soft member and retraction of the same until a position detached from the needle main body.

The core member should be joined to a moving member so as to move the moving member that protrudes from a handle joined to the needle main body, thereby carrying out axial movement of the core member.

### Results of the Invention

The backflush needle of the present invention provides a beneficial result of controlling the soft member, which is connected to the front end of the needle main body, from being deformed and detached when the needle main body is passed through the cannula.

### BRIEF DESCRIPTION OF DRAWINGS

FIG. 1 is a diagram describing a backflush needle of the present invention, wherein FIG. 1(a) is a front view, FIG. 1(b) is an internal structural drawing with a protruding core member, and FIG. 1(c) is an internal structural drawing with a retracted core member;
FIG. 2 is an enlarged view of the tip of the backflush needle of the present invention;
FIG. 3(a) and FIG. 3(b) are diagrams illustrating other moving members; and
FIG. 4 is a use explanation drawing of the backflush needle.

### DESCRIPTION OF EMBODIMENTS

An embodiment according to the present invention is described below with reference to accompanying drawings.

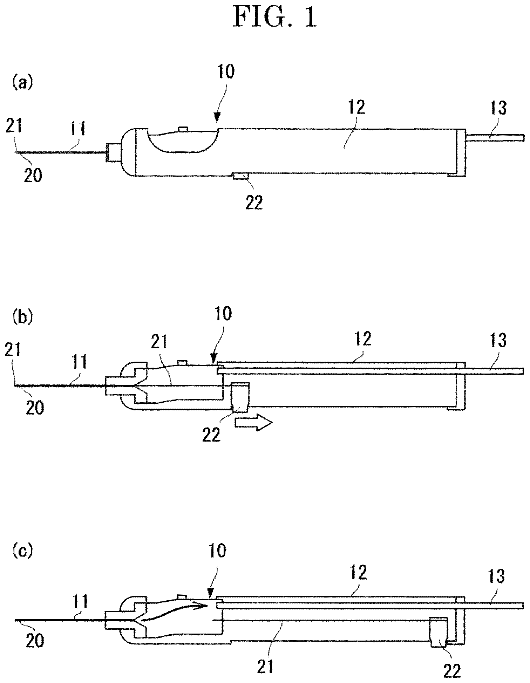

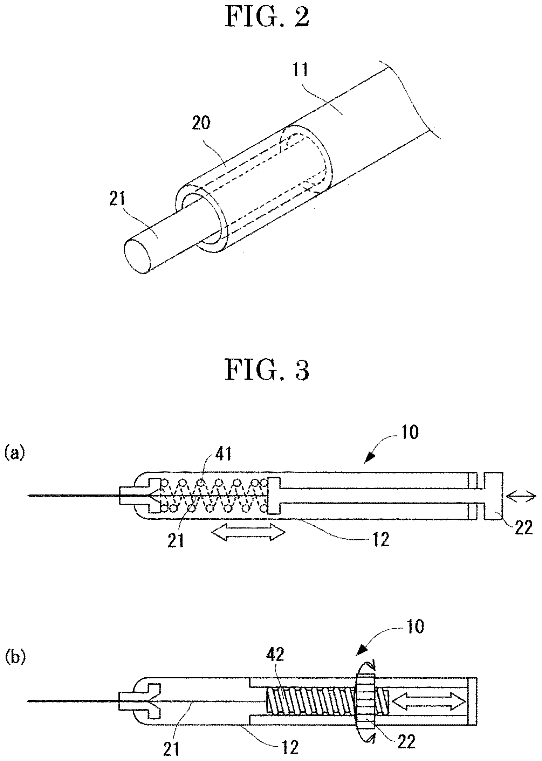

FIG. 1 is a diagram describing a backflush needle of the present invention, wherein FIG. 1(a) is a front view, FIG. 1(b) is an internal structural drawing with a protruding core member, and FIG. 1(c) is an internal structural drawing with a retracted core member. FIG. 2 is an enlarged view of the tip of the backflush needle of the present invention.

The cannula through which the backflush needle 10 is passed is configured in the same manner as the conventional cannula, having a metal cannula pipe fit in a cannula base made of resin. Moreover, a cannula cap is provided for covering the back ends of the cannula base and the cannula pipe in order to control vitreous humor from being discharged via the cannula pipe when the cannula is attached to an eyeball.

The basic structure of the backflush needle 10 is also similar to that of the conventional needle, having a small, tubular needle main body 11 to be connected to a handle 12, which is for a surgeon to grip, and a soft member 20 to be connected to a front end of the needle main body 11. The soft member 20 is formed from silicone resin, it has the same tubular form as that of the needle main body 11, and has approximately the same outer diameter and hollow cavity diameter as those of the needle main body 11. Furthermore, connection between the needle main body 11 and the soft member 20 is also similar to that of the conventional cannula, where it is carried out using an adhesive, and the connecting portion is made to have a fittable structure.

The backflush needle 10 of the present invention has a characteristic of having a core member 21 that passes through the hollow tubular cavities of the needle main body 11 and the soft member 20. While the material of the core member 21 is suitably stainless steel, it is not particularly limited thereto.

The core member 21 is a rod-like member having an outer diameter slightly smaller than the hollow cavity diameters of the needle main body 11 and the soft member 20, and is connected to a moving member 22 that allows moving of the core member 21 from the outside of the handle 12. Note that if the core member 21 is too thin, deformation of the soft member 20 cannot be controlled, and if it has nearly the same outer diameter as the hollow cavity diameters of the needle main body 11 and the soft member 20, movement of the core member 21 forward and backward in the axial direction may be a cause for dull movement of the core member by friction and for catching on the soft member 20, thereby making the soft member 20 fall off from the needle main body 11. Thus, it should have a smaller outer diameter than the hollow cavity diameters of the needle main body 11 and the soft member 20.

The moving member 22 illustrated in FIG. 1 protrudes from a side of the handle 12, moving of the backflush needle 10 axially by pinching the moving member 22 moves the core member 21 axially.

Structure of the moving member may be different from that given in FIG 1. FIG. 3(a) and FIG. 3(b) are diagrams illustrating other moving members. Note that these diagrams simply illustrate the structure of the moving member 22, and unnecessary members are omitted from the description thereof.

FIG. 3(a) illustrates a structure including a spring 41 inside of the handle 12, wherein pushing the button-shaped moving member 22 provided at the back end of the handle 12 makes the core member 21 move forward and backward axially, in the same manner as a structure that pushes in and out a push ballpoint pen core.

FIG. 3(b) illustrates a structure having a spring member 42 that is connected to the core member 21 so as to rotate and move the moving member 22 and thereby moving the spring member 42 axially, which allows axial movement of the core member 21 as well.

Note that the moving member 22 is not limited to that structure, and it may have a different structure as long as the core member 21 can be moved forward and backward axially.

When the core member 21 is pulled out foremost using the moving member 22 (FIG. 1(b)), the front end of the core member 21 should protrude slightly from the front end of the soft member 20 (FIG. 2), and when the core member is pulled out rearmost (FIG. 1(c)), the core member 21 should be at a rearward distance from the needle main body 11.

This is because when the core member 21 is pulled out forward and is protruding from the front end of the soft member 20, there is a result of preventing deformation throughout the entire soft member 20, and if the core member 21 is at a rearward distance from the needle main body 11 when the core member 21 is pulled out rearward, aspirated vitreous body etc. may be passed through the inside of the handle 12 from the hollow cavity of the needle main body 11 so as to lead it to the pipe 13 that is provided on the back end side of the handle 12 (see arrow in FIG. 1(c)).

Use of the backflush needle 10 including the core member 21 as described above prevents deformation of the soft member 20 and prevents the connecting portion of the soft member 20 and the needle main body 11 from falling off as long as the core member 21 is made to protrude from the soft member 20 when passing the soft member 20 and the needle main body 11 through the cannula. Moreover, retraction of the core member 21 during aspiration work allows aspiration of the vitreous body etc. without any problem.

### Explanation of References

10: Backflush needle
11: Needle main body
12: Handle
13: Pipe
20: Soft member
21: Core member
22: Moving member
30: Cannula
31: Cannula pipe
32: Cannula base
33: Cannula cap
41: Spring
42: Screw member
E: Eyeball

## Claims

1. A backflush needle (10) for use used in an ophthalmic operation, wherein the backflush needle (10) is configured to be passed through a cannula (30) attached to an eyeball (E), wherein the backflush needle (10) comprises:
a tubular needle main body (11) configured to pass through the cannula (30),
a tubular soft member (20) formed from silicone resin and connected to a front end of the needle main body (11), and
a rod-shaped core member (21) configured to pass through the tubular hollow cavities of the needle main body (11) and of the soft member (20), respectively,
wherein the core member (21) is axially movable, allowing protrusion of a front end of the core member (21) from the soft member (20) and retraction of the same until a detached position from the needle main body.

2. The backflush needle of Claim 1, wherein the core member (21) is joined to a moving member (22), so as to move the moving member (22) that protrudes from a handle (12) joined to the needle main body (11), thereby carrying out axial movement of the core member (21).

## Patentansprüche

1. Rückspülnadel (10) zur Verwendung bei einer Augenoperation, wobei die Rückspülnadel (10) dazu eingerichtet ist, durch eine an einem Augapfel (E) angebrachte Kanüle (30) geführt zu werden, wobei die Rückspülnadel (10) umfasst:
einen rohrförmigen Nadelhauptkörper (11), der zum Durchtritt durch die Kanüle (30) ausgebildet ist;
ein aus Silikonharz geformtes und mit einem vorderen Ende des Nadelhauptkörpers (11) verbundenes rohrförmiges Weichteil (20); und
ein stabförmiges Kernelement (21), das zum Durchtritt durch die rohrförmigen Hohlräume des Nadelhauptkörpers (11) und des Weichteils (20) jeweils ausgebildet ist,
wobei das Kernelement (21) axial beweglich ist, wodurch das Vorstehen eines vorderen Endes des Kernelements (21) aus dem Weichteil (20) und das Zurückziehen desselben bis zu einer vom Nadelhauptkörper gelösten Position ermöglicht wird.

2. Rückspülnadel nach Anspruch 1, wobei das Kernelement (21) mit einem beweglichen Element (22) verbunden ist, um das von einem mit dem Nadelhauptkörper (11) verbundenen Griff (12) vorstehende bewegliche Element (22) zu bewegen, wodurch eine axiale Bewegung des Kernelements (21) ausgeführt wird.

## Revendications

1. Aiguille d'aspiration (10) destinée à être utilisée dans une opération ophtalmique, dans laquelle l'aiguille d'aspiration (10) est configurée pour être passée à travers une canule (30) fixée à un globe oculaire (E), dans laquelle l'aiguille d'aspiration (10) comprend :
un corps principal d'aiguille tubulaire (11) configuré pour passer à travers la canule (30),
un élément tubulaire souple (20) formé à partir de résine de silicone et relié à une extrémité avant du corps principal d'aiguille (11), et
un élément central en forme de tige (21) configuré pour traverser les cavités tubulaires creuses du corps principal d'aiguille (11) et de l'élément souple (20), respectivement,
dans laquelle l'élément central (21) est mobile axialement, permettant la protrusion d'une extrémité avant de l'élément central (21) à partir de l'élément souple (20) et la rétraction de celui-ci jusqu'à une position détachée du corps principal d'aiguille.

2. Aiguille d'aspiration selon la revendication 1, dans laquelle l'élément central (21) est relié à un élément mobile (22), de manière à déplacer l'élément mobile (22) qui dépasse d'une poignée (12) reliée au corps principal d'aiguille (11), réalisant ainsi un mouvement axial de l'élément central (21).
